# EUROPEAN PATENT SPECIFICATION

(11) **EP 2 990 043 B1**
(45) Date of publication and mention of the grant of the patent: **05.09.2018**
(21) Application number: 14789010.7
(22) Date of filing: 22.04.2014
(51) Int. Cl.: A61K 31/7105, A61K 31/713, A61P 7/10, A61P 25/00, A61P 25/02, A61P 25/28, A61P 27/02, C12N 15/09

(54) **INHIBITORS OF ADAM12 FOR USE IN TREATING NEUROLOGICAL DISORDERS**
INHIBITOREN VON ADAM12 ZUR BEHANDLUNG VON NEUROLOGISCHEN KRANKHEITEN
INHIBITEURS D'ADAM12 POUR LE TRAITEMENT DES MALADIES NEUROLOGIQUES

(30) Priority: 25.04.2013 JP 2013093071
(43) Date of publication of application: 02.03.2016
(73) Proprietor: Yamaguchi University, Yamaguchi 753-8511 (JP)
(72) Inventor: IKEDA, Eiji, Ube-shi Yamaguchi 755-8505 (JP); CUI, Dan, Ube-shi Yamaguchi 755-8505 (JP)
(74) Representative: Script IP Limited
(86) International application number: PCT/JP2014/002259
(87) International publication number: WO 2014/174834

(56) References cited:
- EP-A1- 1 797 897
- WO-A1-2012/005229
- WO-A1-2012/104581
- US-A1- 2009 036 396
- TAKAHIDE KODAMA ET AL.: 'Hito Shinkei Kogashu ni Oite ADAM12 wa Tokuiteki ni Ko Hatsugen shiteiru' NIPPON BYORI GAKKAI KAISHI vol. 92, no. L, 2003, page 199, XP008181332

## Description

### Technical Field

The present invention relates to a therapeutic agent for a neurological disorder containing any one of a functional nucleic acid against ADAM (a disintegrin and metalloproteinase) 12 a functional nucleic acid expression vector containing a DNA encoding the functional nucleic acid and a neutralizing antibody against ADAM12 as an active ingredient. The invention is defined in the claims.

### Background Art

Neural tissues such as the brain and retina in adults are segregated from other tissues by barrier functions (the vascular systems have) between blood and the neural tissue, such as the blood-brain barrier and the blood-retinal barrier, which have been differentiated in a tissue-specific manner. Owing to the barrier functions, an optimal tissue microenvironment is maintained so as for nerve cells to be able to normally function. The vascular barrier functions of the adult neural tissues are induced during the ontogenesis process. The vascular barrier function once induced is not a static trait but is placed under kinetic control. In adults, in order to maintain an optimal microenvironment for normally functioning neural cells, the vascular barrier functions are tactically regulated. In intractable neurological disorders such as diabetic retinopathy, ischemic cerebrovascular disorder and Alzheimer's disease, the disturbance of the tissue microenvironment caused by breakdown of vascular barrier functions works a major factor for exacerbating pathologic conditions. Thus, regulatory factors for vascular barrier functions are expected to be used as a target for developing novel therapies for preventing exacerbation of intractable neurological disorders.

The neural vascular barrier functions are dependent upon tight junctions (hereinafter sometimes referred to as "TJ"), which are formed between endothelial cells of a capillary vessel. Because of this, studying the TJ constituent molecules is a main strategy for elucidating vascular barrier mechanisms. As the TJ constituent molecules, occludins, claudins, which are a family consisting of 27 members, and junctional adhesion molecule A (JAM-A) have been specified. It has been reported that one of the claudin family members expressed and localized in cell membrane of physiological-state neural vascular endothelial cells, namely, claudin-5, is essential for the vascular barrier function (see, non-patent document 1). However, as to control mechanisms for vascular barrier functions, many things still remain unsolved.

Breakdown of a neural vascular barrier allows not only invasion of neurotoxic molecules into tissues but also effusion of plasma components, causing tissue edemas. If edemas persist beyond a predetermined period, irreversible failure occurs in the neural tissues. In current medical care, brain edemas caused by the vascular barrier breakdown are primarily treated by increasing plasma colloid osmotic pressure by intravenous administering Glyceol to allow the interstitial fluid to move into blood vessels. As another idea, administration of corticosteroid is considered as an effective therapy for brain edemas; however, the indication for the therapy thereof is low because the mechanism for mitigating brain edemas is not clear and accompanies many side effects. In the retinal edema, it is reported that intravitreal administration of a neutralizing antibody against a vascular endothelial growth factor (VEGF) having an angiogenic action and a vascular permeability enhancing effect is effective. However, the neutralizing antibody has not been a therapeutic agent directly acting on the blood vessel whose vascular barrier is broken. The anti-VEGF antibody therapy also has a problem of increasing a risk of developing cardiac infarction and brain infarction.

Recently, involvement of ADAM10 and ADAM17 in shedding of JAM-A through inflammatory stimuli (see, non-patent document 2) and involvement of ADAM15 in vascular endothelial cell barrier failure observed in atherosclerosis (see, non-patent document 3) have been proposed; however, association of them with neural vascular barrier breakdown mechanism is neither described nor suggested.

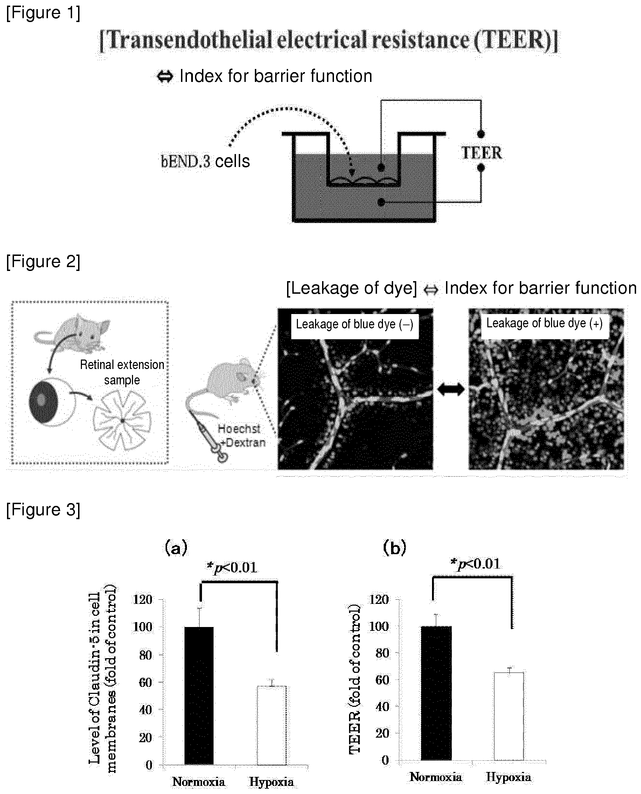

As described above, breakdown of the neural vascular barrier function lies at the center of pathologic conditions of various intractable neurological disorders, and works as a main factor for exacerbating the pathologic conditions; however, the mechanism for breakdown of the vascular barrier function is not known. Elucidation of the breakdown mechanism and development of a therapeutic drug for regulating the vascular barrier function based on the elucidation are anticipated. As an obstacle in promoting studies on the vascular barrier breakdown mechanism, absence of useful experimental systems for specifying factors responsible for the vascular barrier breakdown can be pointed out. In the circumstances, the present inventors noticed hypoxia which presents in a lesion tissue and serves as a trigger of the vascular barrier breakdown, and established *in vitro* and *in vivo* experimental systems narrowed a focus on the hypoxia and reported it (see, non-patent document 4). As described in non-patent Document 4, in the *in vitro* system, a mouse brain endothelial cell line (bEND. 3 cells) was used and electrical resistance (TEER) of the cellular monolayer was used as an index for the vascular barrier, as shown in Figure 1; whereas in the *in vivo* system, mice were raised at a low oxygen concentration and the permeability of the blood vessel within the retinal tissue placed in a hypoxic metabolic state was used as an index for the vascular barrier, as shown in Figure 2. Using the above experimental systems, the inventors found a cascade of events: (1) emergence of a hypoxia region in the neural tissue of an intractable neurological disorder, (2) disappearance of claudin-5 from cell membrane of vascular endothelial cells, (3) breakdown of a vascular barrier function and (4) exacerbation of pathologic condition.

### Prior Art Documents

### Non-patent Documents

Non-patent Document 1: Nitta T, et al. J. Cell Biol.(2003) 161:653-660
Non-patent Document 2: Koenen RR, et al. Blood (2009) 113:4799-4809
Non-patent Document 3: Sun C, et al. Arterioscler Thromb Vasc Biol. (2012) 32:2444-2451
Non-patent Document 4: Koto T, et al. Am. J. Pathol. (2007) 170:1389-1397

### Summary of the Invention

### Object to be Solved by the Invention

The neurological disorder therapeutic agents currently known are not those directly acting on the blood vessel itself whose vascular barrier is broken down, more specifically, are not those directly acting on the substance expressed in the vascular endothelial cells constituting a blood vessel, and accompany many side effects. For the reasons, indication for therapy of the therapeutic agents was low. In the circumstances, an object of the present invention is to provide a neurological disorder therapeutic agent directly acting on oxygen concentration-dependent vascular barrier breakdown.

### Means to Solve the Object

In the circumstance where established therapies for neurological disorders caused by vascular barrier breakdown are absent, the present inventors have conducted studies with a view to specifying a factor responsible for vascular barrier breakdown in intractable neurological disorders and developing a specific therapeutic drug targeting the factor. The inventors first narrowed a focus of study on analyzing a mechanism underlying "disappearance of claudin-5 from cell membrane of vascular endothelial cells by hypoxic stimulation" for specifying a factor responsible for vascular barrier breakdown. During the process of this study, we found that the ubiquitin-proteasome system works in disappearance of claudin-5 from cell membrane; however, it revealed that the activity thereof does not depend upon the oxygen concentration. We determined that the ubiquitin-proteasome system is involved in turnover of claudin-5 in the physiological state and not involved in vascular barrier breakdown mechanism induced specifically to a pathological condition of a neurological disorder. Then, we remade a strategy towards specifying a mechanism underlying disappearance of claudin-5 from cell membrane of endothelial cells depending upon oxygen concentration. We exposed bEND. 3 cells to hypoxia *in vitro* and observed expression and localization of claudin-5 over time. As a result, the inventors showed that claudin-5, which is localized in cell membrane under normoxia, disappears from the cell membrane in as a short a time as 30 minutes after the cells are exposed to hypoxia, and that the amount of claudin-5 protein in the entire cell reduces. Considering these findings all together, we postulated that the factor responsible for disappearance of claudin-5 from cell membrane of vascular endothelial cells under hypoxia can be a proteolytic enzyme already expressed in the vascular endothelial cells even under normoxia and localized in the cell membrane. As a candidate for the enzyme falling under the postulation, members of an ADAM family were suggested. Then, for specifying a responsible factor, studies were conducted using the *in vitro* experimental system (described in non-patent document 4) established by the inventors; at the same time, for evaluating the efficiency of the factor as a target for treating a pathological vascular barrier breakdown, studies were conducted using *in vivo* experimental system. As a result, ADAM12 and ADAM17 expressing in vascular endothelial cells are specified as the factors responsible for causing vascular barrier breakdown in neurological disorders and found to be useful also as a therapeutic target. Furthermore specific siRNA against genes of ADAM12 and ADAM17 (which were specified as factors responsible for vascular barrier breakdown) and antibodies against ADAM12 and ADAM17 were intravitreally administered. As a result, hyper permeability of the retinal vessel was found to be drastically improved. Moreover, ADAM17 knockout mice were placed under hypoxia. As a result, an increase of fluorescent dye leakage from the blood vessel in the retinal tissue was not observed. From this, we found that oxygen concentration-dependent vascular barrier breakdown can be suppressed by suppressing expression of the ADAM17 gene. Based on the findings, the present invention was accomplished.

More specifically, the present disclosure relates to,
[1] A therapeutic agent for a neurological disorder containing, as an active ingredient, any one of (a) a functional nucleic acid against ADAM (a disintegrin and metalloproteinase) 12 or ADAM17, (b) a functional nucleic acid expression vector containing a DNA encoding the functional nucleic acid (a) and (c) a neutralizing antibody against ADAM12 or ADAM17;
[2] The therapeutic agent for a neurological disorder according to [1], wherein the functional nucleic acid is siRNA;
[3] The therapeutic agent for a neurological disorder according to [2], wherein the functional nucleic acid against ADAM12 is a siRNA consisting of a sense strand sequence of nucleotides represented by SEQ ID NO: 1 and an antisense strand sequence complementary thereto represented by SEQ ID NO: 2, or a siRNA consisting of a sense strand sequence of nucleotides represented by SEQ ID NO: 3 and an antisense strand sequence complementary thereto represented by SEQ ID NO: 4;
[4] The therapeutic agent for a neurological disorder according to [2], wherein the functional nucleic acid against ADAM17 is a siRNA consisting of a sense strand sequence of nucleotides represented by SEQ ID NO: 5 and an antisense strand sequence complementary thereto represented by SEQ ID NO: 6, or a siRNA consisting of a sense strand sequence of nucleotides represented by SEQ ID NO: 7 and an antisense strand sequence complementary thereto represented by SEQ ID NO: 8;
[5] The therapeutic agent for a neurological disorder according to any one of [1] to [4], wherein the neurological disorder is a cerebral neurological disorder or a retinal neurological disorder;
[6] The therapeutic agent for a neurological disorder according to [5], wherein the cerebral neurological disorder is brain edema; and
[7] The therapeutic agent for a neurological disorder according to [5], wherein the retinal neurological disorder is retinal edema.

### Effect of the Invention

According to the present invention, since oxygen concentration-dependent vascular barrier breakdown can be directly suppressed, a neurological disorder can be effectively treated with few side effects.

### Brief Description of Drawings

[Figure 1] Figure 1 shows an *in vitro* system using a mouse brain endothelial cell line (bEND. 3) and employing the electrical resistance (TEER) of cell monolayer thereof as index for barrier.
[Figure 2] Figure 2 shows an *in vivo* system using the permeability of the blood vessel within the retinal tissue (which is placed in a hypoxic metabolic state by raising mice at a low oxygen concentration) as an index for the vascular barrier. In the blood vessel within the retinal tissue placed in a hypoxic metabolic state, leakage of the fluorescent dye injected as a tracer was increased (right photograph); however, leakage of the fluorescent dye was suppressed in the mice raised under normoxia (left photograph).
[Figure 3] Figure 3 shows the expression level of claudin-5 in cell membrane of an endothelial cell line derived from a mouse brain blood vessel, which were obtained by culturing at a confluent state for 5 days under normoxia and placing under hypoxia for 30 minutes (Figure 3(a)); and the measurement results of electrical resistance (TEER) of cell monolayer (Figure 3(b)). Black columns show the results under normoxia; whereas white columns show the results under hypoxia; and the ordinate axis represents relative values based on the value under normoxia as 100. The p value was lower than a standard value (p = 0.05) based on which significant difference judgment is made.

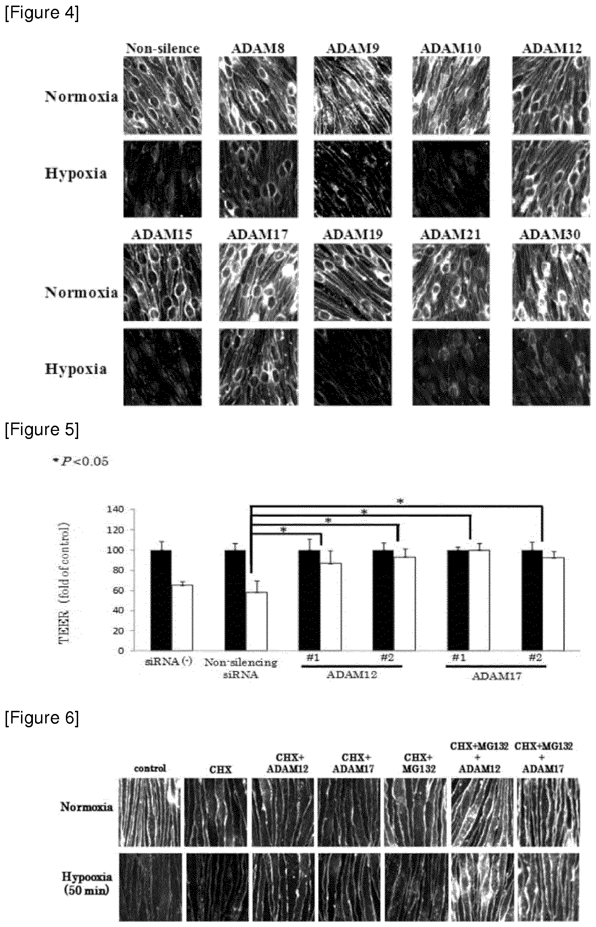

[Figure 4] Figure 4 shows the fluorescent observation results of expression and localization of claudin-5 under normoxia and hypoxia 2 days after siRNA No. ADAM8#1, No. ADAM9#1, No. ADAM10#1, No. ADAM12#1, No. ADAM15#1, No. ADAM17#1, No. ADAM19#1, No. ADAM21#1 and No. ADAM30#1 were separately introduced into bEND. 3 cells.
[Figure 5] Figure 5 shows the measurement results of TEER of bEND. 3 cells having siRNA No. ADAM12#1, #2 and No. ADAM17#1 and #2 separately introduced thereinto and obtained by culturing under normoxia and hypoxia for 30 minutes. Black columns show the results under normoxia; whereas white columns show the results under hypoxia, and the ordinate axis represents relative values based on the value in the case where a non-silencing siRNA was introduced under normoxia as 100.
[Figure 6] Figure 6 shows immunostaining results of expression of claudin-5 in bEND. 3 cells having siRNA introduced thereinto, which were obtained by culturing under hypoxia (1% oxygen concentration) for 50 minutes in the presence of a protein translation inhibitor, cycloheximide (CHX) or in the presence of CHX and an inhibitor for a ubiquitin - proteasome system, MG132. Control indicates the culture results of bEND. 3 cells having non-silencing siRNA introduced thereinto; CHX indicates the culture results of bEND. 3 cells having non-silencing siRNA introduced thereinto in the presence of CHX; CHX + ADAM12 indicates the culture results of bEND. 3 cells having siRNA No. ADAM12#1 introduced thereinto in the presence of CHX; CHX + ADAM17 indicates the culture results of bEND. 3 cells having siRNA No. ADAM17#1 introduced thereinto in the presence of CHX; CHX + MG132 indicates the culture results of bEND. 3 cells having a non-silencing siRNA introduced thereinto in the presence of CHX and MG132; CHX + MG132 + ADAM12 indicates the culture results of bEND. 3 cells having siRNA No. ADAM12#1 introduced thereinto in the presence of CHX and MG132; and CHX + MG132 + ADAM17 indicates the culture results of bEND. 3 cells having siRNA No. ADAM17#1 introduced thereinto in the presence of CHX and MG132.

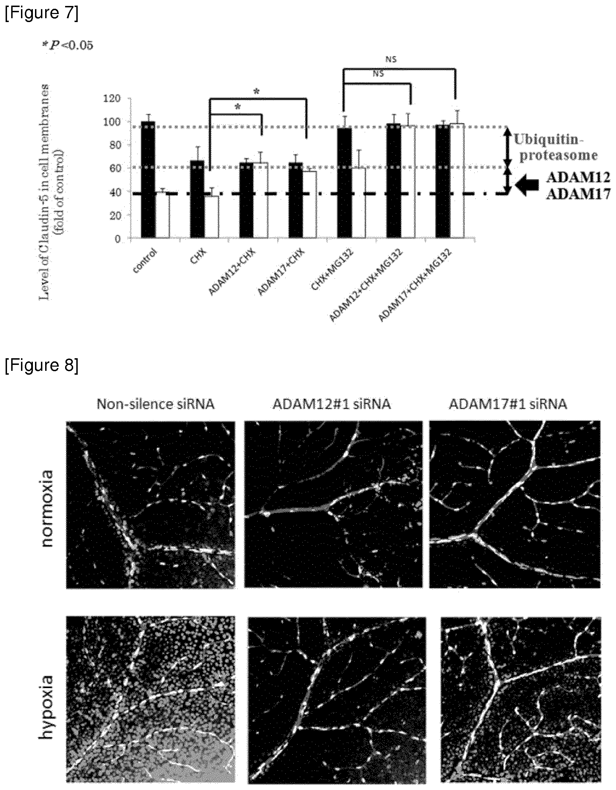

[Figure 7] Figure 7 is a graph quantitatively showing the immunostaining results of Figure 6. Black columns show normoxia; whereas white columns show hypoxia. The ordinate axis represents relative values based on the value of control under normoxia as 100. The p value was lower than a standard value (p = 0.05) based on which significant difference judgment was made.
[Figure 8] Figure 8 shows confocal microscopic observation results of leakage of the tracer injected in mice raised under normoxia for 48 hours (upper stage: normoxia) and mice raised under hypoxia of 4% to 7% for 48 hours (lower stage: hypoxia) after siRNA No. ADAM12#1 and No. ADAM17#1 were intravitreally administered to the mice.

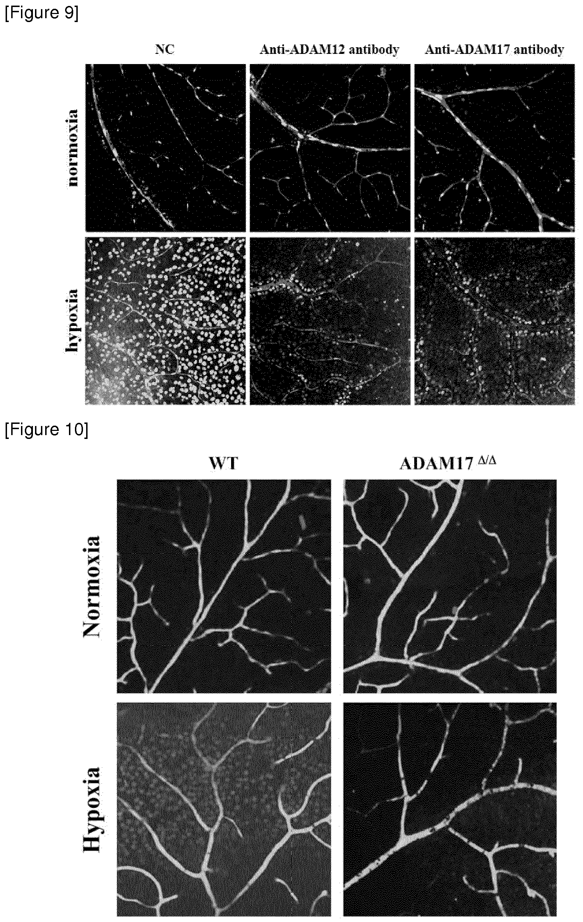

[Figure 9] Figure 9 shows confocal microscopic observation results of leakage of the tracer injected in mice raised under normoxia for 24 hours (upper stage: normoxia) and mice raised under hypoxia of 4% to 7% for 24 hours (lower stage: hypoxia) after normal goat serum-derived IgG (left row: NC), anti-ADAM12 antibody (goat anti-human ADAM12 polyclonal antibody (center row: Anti-ADAM12 antibody)) and anti-ADAM17 antibody (goat anti-human ADAM17 polyclonal antibody (right row: Anti-ADAM17 antibody)) were intravitreally administered to the mice.
[Figure 10] Figure 10 shows confocal microscopic observation results of leakage of the tracer injected in wild type mice (left row: WT) and ADAM17 knockout mice (right row: ADAM17^{Δ/Δ}) raised under normoxia for 48 hours (upper stage: normoxia) and under hypoxia of 4% to 7% for 48 hours (lower stage: hypoxia).

### Mode of Carrying Out the Invention

The neurological disorder therapeutic agent according to the present invention is not particularly limited as long as it is a therapeutic agent for a neurological disorder (hereinafter, sometimes referred to as "the neurological disorder therapeutic agent of the present invention") containing, as an active ingredient, any one of (a) a functional nucleic acid against ADAM (a disintegrin and metalloproteinase) 12 or ADAM17, (b) a functional nucleic acid expression vector containing a DNA encoding the functional nucleic acid (a) and (c) a neutralizing antibody against ADAM12 or ADAM17. The neurological disorder therapeutic agent of the present invention can repair the neural vascular barrier function itself by suppressing disappearance of claudin-5 depending upon the oxygen concentration, thereby effectively treating a neurological disorder with few side effects.

Other embodiments of the neurological disorder therapeutic agent include a method for treating a neurological disorder by administering, to a subject, any one of (a) functional nucleic acid against ADAM12 or ADAM17; (b) a functional nucleic acid expression vector containing a DNA encoding the functional nucleic acid (a); and (c) a neutralizing antibody against ADAM12 or ADAM17, and any one of (a) functional nucleic acid against ADAM12 or ADAM17; (b) a functional nucleic acid expression vector containing a DNA encoding the functional nucleic acid (a); and (c) a neutralizing antibody against ADAM12 or ADAM17 for the use as a neurological disorder therapeutic agent, and the use of any one of (a) functional nucleic acid against ADAM12 or ADAM17; (b) a functional nucleic acid expression vector containing a DNA encoding the functional nucleic acid (a); and (c) a neutralizing antibody against ADAM12 or ADAM17 in preparing a neurological disorder therapeutic agent.

ADAM12 and ADAM17 are metalloproteases belonging to the ADAM family, expressing in vascular endothelial cells, and known to cleave, e.g., a ligand for a receptor-type tyrosine kinase of an extracellular domain of a membrane-bound protein and TNF-receptor ligand TNF-α, and to be involved in cancer, vascular disorders and inflammatory disorders such as rheumatoid arthritis.

The functional nucleic acid against ADAM12 or ADAM17 can be siRNA, antisense RNA, miRNA, shRNA, ribozyme or aptamer to ADAM12 or ADAM17; more specifically, siRNA, antisense RNA, miRNA, shRNA or ribozyme which suppresses expression of an ADAM12 gene or an ADAM17 gene, and an aptamer inactivating or reducing the physiological activity of ADAM12 or ADAM17; and preferably, siRNA against the ADAM12 gene or the ADAM17 gene. In order to more effectively suppress vascular barrier breakdown, siRNA against the ADAM12 gene is more preferable. The functional nucleic acids can be designed, for example, by methods described in the following documents (Wadhwa, R, et al. Reviews in Mutat. Res. (2004) 567:71-84, Wadhwa, R. et al. Current Opinions in Molecular Therapeutics. (2004) 6:367-372, Wadhwa, R. et al. EMBO (2003) 4:595-601).

The siRNA against the ADAM12 gene or ADAM17 gene consists of a sense strand sequence of nucleotides which is homologous to mRNA of the ADAM12 gene or ADAM17 gene and an antisense strand sequence complementary thereto, and refers to a double-stranded RNA suppressing expression of the ADAM12 gene or ADAM17 gene. The double-stranded RNA preferably has a length of 17 to 27 base pairs and more preferably a length of 18 to 20 base pairs. The siRNA against the ADAM12 gene is preferably a siRNA consisting of a sense strand sequence of nucleotides represented by SEQ ID NO: 1 and an antisense strand sequence complementary thereto represented by SEQ ID NO: 2; or a siRNA consisting of a sense strand sequence of nucleotides represented by SEQ ID NO: 3 and an antisense strand sequence complementary thereto represented by SEQ ID NO: 4. The siRNA against the ADAM17 gene can be a siRNA consisting of a sense strand sequence of nucleotides represented by SEQ ID NO: 5 and an antisense strand sequence complementary thereto represented by SEQ ID NO: 6 or preferably a siRNA consisting of a sense strand sequence of nucleotides represented by SEQ ID NO: 7 and an antisense strand sequence complementary thereto represented by SEQ ID NO: 8. The expression suppressive action of the ADAM12 gene or ADAM17 gene can be enhanced by adding an overhang sequence or preferably adding any two bases of thymine, guanine, cytosine and adenine to the 3' side of each of the strands.

The sense strand sequence homologous to mRNA of the ADAM12 gene or ADAM17 gene and the antisense strand sequence complementary thereto can be designed based on the sequence information of the ADAM12 gene or ADAM17 gene, in accordance with e.g., methods described in the following documents (Wadhwa, R, et al. Reviews in Mutat. Res. (2004) 567:71-84, Wadhwa, R. et al. Current Opinions in Molecular Therapeutics. (2004) 6:367-372), and prepared by a synthesis method and a method using a gene recombination technology.

The functional nucleic acid expression vector containing a DNA encoding the functional nucleic acid is not particularly limited as long as it is a vector capable of expressing the functional nucleic acid. For example, if the functional nucleic acid is siRNA, the functional nucleic acid expression vector can be prepared by inserting a double-stranded RNA expression cassette, which consists of a sense strand sequence of specific nucleotides of the ADAM12 gene or ADAM17 gene, a linker and an antisense strand sequence complementary thereto, downstream a promoter. The vector for use in preparing such a functional nucleic acid expression vector can be a vector known in the art including a commercially available vector. However, when the vector is introduced into a mammal, a viral vector is preferable. Specific examples of the viral vector include a mouse leukemia retroviral vector (Microbiology and Immunology (1992) 158, 1-23), an adeno-associated viral vector (Muzyczka N. Curr. Top. Microbiol. Immunol. (1992) 97-129), an adenovirus vector (Rosenfeld M, et al. Science (1991) 252, 431-434) and a liposome. HIV lentiviral vector is preferable for the reason that it can be efficiently expressed for a long period even in non-dividing cells. Furthermore, these expression systems may contain a control sequence regulating the expression other than the sequences involved in inducing expression.

In the present disclosure a neutralizing antibody against ADAM12 or ADAM17 refers to an antibody, which specifically binds to ADAM12 or ADAM17 to inactivate or reducing the physiological activity of ADAM12 or ADAM17. Such a neutralizing antibody can be more preferably a neutralizing antibody against ADAM12 in order to more suppress vascular barrier breakdown.

The above neutralizing antibody can be any type of antibody; examples thereof include a human antibody, a chimeric antibody or a humanized antibody. Other than these, an antibody fragment such as F(ab')2, Fab, diabody, Fv, ScFv or Sc(Fv)2 can be mentioned. Furthermore, the neutralizing antibodies can be polyclonal antibodies or monoclonal antibodies.

The neutralizing antibody can be not only a commercially available anti-ADAM12 antibody or anti-ADAM17 antibody but also an antibody prepared in accordance with a conventional protocol. For example, the human antibody can be prepared by a hybridoma method or a phage display method; whereas, the chimeric antibody or humanized antibody can be prepared in accordance with conventional genetic engineering method. The antibody fragment can be prepared by digesting a full length antibody with pepsin or papain.

In the present invention, the neurological disorder is a disorder caused by breakdown of a neural vascular barrier, and preferably a disorder caused by disturbance of a tissue microenvironment caused by breakdown of a vascular barrier function, more preferably a cerebral neurological disorder such as brain edema, brain infarction, vascular dementia, ischemic cerebrovascular disorder or Alzheimer's disease; or a retinal neurological disorder such as retinal edema or diabetic retinopathy, and particularly preferably, brain edema or retinal edema. Note that the neural blood vessel refers to the capillary of the neural tissue and does not include blood vessels of tissues except the neural tissue.

In the present invention, the neural vascular barrier refers to a mechanism of limiting exchange of substances between blood and tissue fluid of the neural and can favorably refer to a blood-brain barrier limiting exchange of substances between blood and tissue fluid of the brain or a blood-retinal barrier limiting exchange of substances between blood and tissue fluid of the retina.

In the present invention, the phrase "vascular barrier function is broken" means, for example, that mechanism limiting exchange of substances between blood and tissue fluid of the neural does not function, with the result that neurotoxic molecules invade into the tissue and effusion of plasma components occurs.

The neurological disorder therapeutic agent is satisfactory as long as it contains a functional nucleic acid against ADAM12 or ADAM17, the functional nucleic acid expression vector containing a DNA encoding the functional nucleic acid or a neutralizing antibody to ADAM12 or ADAM17 as an active ingredient, and optionally contains pharmaceutically acceptable additives usually used for producing a preparation, such as an excipient, a binder, a lubricant, a disintegrator, an antiseptic agent, an isotonic agent, a stabilizer, a dispersant, an antioxidant, a colorant, a flavor and a buffer. The dosage form of the preparation can be a solid preparation such as a powder and grain, and preferably, a liquid such as a solution, an emulsion and a suspension in order to obtain an excellent therapeutic effect for neurological disorders.

A method for administering the neurological disorder therapeutic agent is not particularly limited as long as a desired therapeutic effect for a neurological disorder can be obtained. Examples of the method include intravenous administration, oral administration, intramuscular administration, subcutaneous administration, transdermal administration, transnasal administration, and transpulmonary administration. Particularly for a retinal neurological disorder, intravitreal administration can be mentioned. The dosage of the neurological disorder therapeutic agent of the present invention is not particularly limited and can be appropriately adjusted depending upon the physical condition, disease condition, body weight, age and gender of a subject such as a human and an animal. The dosage can be, for example, 0.01 µg to 100 g/kg body weight per day, more preferably 0.1 µg to 10 g/kg body weight per day, and further preferably 1 µg to 1 g/kg body weight per day. The neurological disorder therapeutic agent of the present invention can be used in combination with other neurological disorder therapeutic agents. The administration time, period and others of the neurological disorder therapeutic agent of the present invention are not particularly limited. The dosage per day can be administered at a time or several times by dividing the dosage in several portions. The organisms including cells and tissues to be administered are not particularly limited, and can be preferably a mammalian such as a human, a monkey, a bovine, an equine, a sheep, a swine, a canine, a feline, a rat, a mice and a hamster. Of them, a human is preferable.

### Example 1

### (Disappearance of claudin-5 under hypoxia)

An endothelial cell line (bEND. 3 cells) derived from a mouse brain blood vessel was cultured under normoxia (oxygen concentration: 21%) for 5 days in a confluent state and then placed under hypoxia (oxygen concentration: 1%) for 30 minutes. Thereafter, the expression level of claudin-5 on cell membrane and electrical resistance (TEER) of a cell monolayer were measured in accordance with the method of Koto T et al. (Am. J. Pathol. (2007) 170:1389-1397). The results are shown in Figure 3. As shown in Figure 3 (a), it was found that expression level of claudin-5 in bEND. 3 cells 30 minutes after hypoxia reduces by about 40%. As is apparent from Figure 3 (b), the TEER of the cell monolayer in bEND. 3 cells 30 minutes after hypoxia reduces by about 35%.

### Example 2

### (Search for ADAM family member involved in disappearance of claudin-5)

The expression of ADAM family members in bEND. 3 cells under normoxia was analyzed by RT-PCR. As a result, ADAM20, 28, 33 did not express in bEND. 3 cells under normoxia; however, ADAM8, 9, 10, 12, 15, 17, 19, 21 and 30 expressed in bEND. 3 cells under normoxia (data are not shown). Thus, ADAM8, 9, 10, 12, 15, 17, 19, 21 and 30 were used as candidates of a factor involved in disappearance of claudin-5 from cell membrane in response to a reduction of the oxygen concentration. Then 2 types of siRNA against each of the genes of ADAM family members (ADAM8, 9, 10, 12, 15, 17, 19, 21, 30) were prepared and introduced into the endothelial cell line (bEND. 3 cells) derived from a mouse brain blood vessel and cultured in a confluent state for 5 days. The numbers of each siRNA prepared and their sense strand sequences of the nucleotides and antisense strand sequences complementary thereto are shown in Table 1. The siRNA against ADAM8 gene are No. ADAM8#1 and No. ADAM8#2; the siRNA against ADAM 9 gene are No. ADAM9#1 and No. ADAM9#2; the siRNA against ADAM 10 gene are No. ADAM10#1 and No. ADAM10#2; the siRNA against ADAM 12 gene are No. ADAM12#1 and No. ADAM12#2; the siRNA against ADAM 15 gene are No. ADAM15#1 and No. ADAM15#2; the siRNA against ADAM 17 gene are No. ADAM17#1 and No. ADAM17#2; the siRNA against ADAM 19 gene are No. ADAM19#1 and No. ADAM19#2; the siRNA against ADAM 21 gene are No. ADAM21#1 and No. ADAM21#2; and the siRNA against ADAM 30 gene are No. ADAM30#1 and No. ADAM30#2. Note that nucleotide sequences represented by small letters in the table are overhang sequences.

**[Table 1]**

| **siRNA No.** | **Gene Symbol** | **Sense siRNA Sequence** | **Antisense siRNA Sequence** |
|---|---|---|---|
| **ADAM8#1** | Adam8 | **GGAACUCAGUUUUCGAGUUtt SEQ ID No: 9** | **AACUCGAAAACUGAGUUCCtg SEQ ID No: 10** |
| **ADAM8#2** | Adam8 | **GGAACUCAGUUUUCGAGUUtt SEQ ID** No: 11 | **AACUCGAAAACUGAGUUCCtg SEQ ID No: 12** |
| **ADAM9#1** | Adam9 | **CAGAAAUCCUAUCAAUAUAtt SEQ ID No: 13** | **UAUAUUGAUAGGAUUUCUGtc SEQ ID No: 14** |
| **ADAM9#2** | Adam9 | **CGUCCAUUGUUGCUCAUGAtt SEQ ID No: 15** | **UCAUGAGCAACAAUGGACGca SEQ ID No: 16** |
| **ADAM10#1** | Adam10 | **GACUUCUCCGGAAUCCGUAtt SEQ ID No: 17** | **UACGGAUUCCGGAGAAGUCtg SEQ ID No: 18** |
| **ADAM10#2** | Adam10 | **CAUUACUGUUCAGAACUAUtt SEQ ID No: 19** | **AUAGUUCUGAACAGUAAUGat SEQ ID No: 20** |
| **ADAM12#1** | Adam12 | **GCUACCACCUGUACUCUGAtt SEQ ID No: 21** | **UCAGAGUACAGGUGGUAGCgt SEQ ID No: 22** |
| **ADAM12#2** | Adam12 | **CCCUUAAGAUGACCAAGUAtt SEQ ID No: 23** | **UACUUGGUCAUCUUAAGGGtc SEQ ID No: 24** |
| **ADAM15#1** | Adam15 | **GAUGGACCCUGUUGUCAAAtt SEQ ID No: 25** | **UUUGACAACAGGGUCCAUCag SEQ ID No: 26** |
| **ADAM15#2** | Adam15 | **CAGCACAACCUGAUAGAAAtt SEQ ID No: 27** | **UUUCUAUCAGGUUGUGCUGgg SEQ ID No: 28** |
| **ADAM17#1** | Adam17 | **GGACGUAAUUGAGCGAUUUtt SEQ ID No: 29** | **AAAUCGCUCAAUUACGUCCtg SEQ ID No: 30** |
| **ADAM17#2** | Adam17 | **CCAGGAUUUUGAUAUGGGAtt SEQ ID No: 31** | **UCCCAUAUCAAAAUCCUGGta SEQ ID No: 32** |
| **ADAM19#1** | Adam19 | **CUCCUUCUUUGAGACGGAAtt SEQ ID No: 33** | **UUCCGUCUCAAAGAAGGAGgt SEQ ID No: 34** |
| **ADAM19#2** | Adam19 | **CCAACGCAGUAUCUAUUGAtt SEQ ID No: 35** | **UCAAUAGAUACUGCGUUGGat SEQ ID No: 36** |
| **ADAM21#1** | Adam21 | **GCGCAUCUCAUAAUUGCUAtt SEQ ID No: 37** | **UAGCAAUUAUGAGAUGCGCtt SEQ ID No: 38** |
| **ADAM21#2** | Adam21 | **GCCCAUAUCUUCAUUAGAAtt SEQ ID No: 39** | **UUCUAAUGAAGAUAUGGGCtg SEQ ID No: 40** |
| **ADAM30#1** | Adam30 | **GGAGGCUUUUGUUUCCGUAtt SEQ ID No: 41** | **UACGGAAACAAAAGCCUCCgt SEQ ID No: 42** |
| **ADAM30#2** | Adam30 | **GAAGUACUUUCAACGACUAtt SEQ ID No: 43** | **UAGUCGUUGAAAGUACUUCta SEQ ID No: 44** |

Two dishes were prepared for each bEND. 3 cells having individual siRNA introduced thereinto. Two days after introduction of siRNA, one of the two dishes was cultured under normoxia and the other dish was cultured under hypoxia (1% oxygen concentration) for 30 minutes. Thereafter, expression and localization of claudin-5 was checked by immunostaining using a Rabbit anti-mouse claudin-5 (manufactured by Zymed Laboratories) as a primary antibody and AlexaFluor (registered trade mark) 488 Gout anti-Rabbit IgG antibody (manufactured by Invitrogen) as a secondary antibody. Expression and localization of claudin-5 under normoxia and hypoxia in bEND. 3 cells, into which non-silence siRNA, siRNA No. ADAM8#1, No. ADAM9#1, No. ADAM10#1, No. ADAM12#1, No. ADAM15#1, No. ADAM17#1, No. ADAM19#1, No. ADAM21#1 and No. ADAM30#1 were separately introduced, were checked by fluorescent observation 2 days after the introduction. The results are shown in Figure 4. As shown in Figure 4, in bEND. 3 cells into which siRNA No. ADAM12#1 and siRNA No. ADAM17#1 were separately introduced, disappearance of claudin-5 from cell membrane caused by hypoxia was suppressed. Note that the same results were obtained in the bEND. 3 cells, into which siRNA No. ADAM8#2, No. ADAM9#2, No. ADAM10#2, No. ADAM12#2, No. ADAM15#2, No. ADAM17#2, No. ADAM19#2, No. ADAM21#2 and No. ADAM30#2 were separately introduced (the results are not shown in the figure). Furthermore, TEER of bEND. 3 cells having siRNA No. ADAM12#1, #2 and siRNA No. ADAM17#1, #2 each introduced thereinto and cultured under hypoxia (1% oxygen concentration) for 30 minutes, was measured by the same method as in Example 1. The results are shown in Figure 5. As shown in Figure 5, a reduction of TEER in the bEND. 3 cells having siRNA No. ADAM12#1, #2 and siRNA No. ADAM17#1, #2 separately introduced thereinto was suppressed.

From the above results, it was found that ADAM12 and ADAM17 are involved in disappearance of claudin-5, and that vascular barrier breakdown was suppressed by suppressing expression of ADAM12 and ADAM17 genes by use of siRNA against ADAM12 and ADAM17 genes.

### Example 3

### (Examination of ADAM12 and ADAM17 being factors responsible for vascular barrier breakdown under hypoxia)

The bEND. 3 cells having siRNA No. ADAM12#1, 12#2 and No. ADAM17#1, 17#2 introduced thereinto were cultured in the presence of a protein translation inhibitor, i.e., cycloheximide (CHX) or in the presence of cycloheximide and a ubiquitin - proteasome system inhibitor, i.e., MG132, and under hypoxia (1% oxygen concentration) for 50 minutes, and thereafter expression of claudin-5 was checked. The immunostaining results of the bEND. 3 cells having siRNA No. ADAM12#1 and No. ADAM17#1 introduced thereinto are shown in Figure 6; and a graph quantitatively showing the immunostaining results are shown in Figure 7. The antibodies used in immunostaining are the same as in Example 2. As is apparent from Figures 6 and 7, the expression level of claudin-5 in the bEND. 3 cells having siRNA No. ADAM12#1 and No. ADAM17#1 introduced thereinto, treated with CHX and cultured under hypoxia was the same as that under a normal oxygen concentration. Furthermore, the expression level of claudin-5 in the bEND. 3 cells having siRNA No. ADAM12#1 and No. ADAM17#1 introduced thereinto, treated with CHX and MG132 and cultured under hypoxia was the same as not only that under a normal oxygen concentration but also that in the control where the cells were cultured under a normal oxygen concentration without CHX treatment. The same results were obtained in bEND. 3 cells having siRNA No. ADAM12#2 and No. ADAM17#2 introduced thereinto (the results are not shown in Figure). It was confirmed that the ubiquitin - proteasome system is involved in disappearance of claudin-5 from the cell membrane of bEND. 3 cells, at a normal oxygen concentration; whereas, ADAM12 and ADAM17 are involved in addition to the ubiquitin - proteasome system in the disappearance at a low oxygen concentration as responsible molecules. It was further confirmed that disappearance of claudin-5 is suppressed by using siRNA against ADAM12 and ADAM17 genes and thereby vascular barrier breakdown is suppressed.

### Example 4

### (Examination of ADAM12 and ADAM17 as therapeutic target)

Usefulness of ADAM12 and ADAM17 as a therapeutic target was examined by use of the *in vivo* experimental system. Using fluorescent dyes, Tetramethylrhodamine-conjugated lysine fixable dextran (10kDa; manufactured by Invitrogen) and Hoechst stain H33252 (534Da; manufactured by Invitrogen) as a tracer, and the permeability (used as an index for blood-retinal barrier function) of the retinal vessel was evaluated. siRNA No. ADAM12#1, 12#2 and No. ADAM17#1, 17#2 (each in a final concentration: 250 nM) each were intravitreally administered to two mice. One of the two mice was raised under normoxia for 48 hours, whereas the other mouse was raised under hypoxia of 4% to 7% for 48 hours. The fact that the retinal tissue of the mouse raised under hypoxia is in a hypoxic metabolic state was confirmed by administration of pimonidazole. The above two types of fluorescent dyes were administered in the heart of each of the mice and thereafter retinal extension samples were prepared. Leakage of the tracer injected was evaluated by a confocal microscope. The confocal microscopic observation results in the cases where siRNA No. ADAM12#1 and siRNA No. ADAM17#1 were separately administered to mice intravitreally, are shown in Figure 8. As shown in Figure 8, in the mouse to which a control (Non-silence siRNA) was administered, the retinal vascular barrier was broken under hypoxia, and leakage of fluorescent dyes intravenously administered increased. In contrast, in mice where siRNA No. ADAM12#1 and No. ADAM17#1 were separately administered intravitreally, leakage of fluorescent dyes can be suppressed also in a low-oxygen environment and permeability was recovered to the same level as that of the retinal vessel of the mouse raised under normoxia. The same results were obtained in mice where siRNA No. ADAM12#2 and No. ADAM17#2 were separately administered (the results are not shown in the figure). Particularly, leakage of fluorescent dyes was more efficiently suppressed in the mouse, to which siRNA No. ADAM12#1 or siRNA No. ADAM12#2 was administered, than that of the mouse, to which siRNA No. ADAM17#1 or siRNA No. ADAM17#2 was administered. From the above results, it was demonstrated that siRNA against ADAM12 gene or ADAM17 gene is useful as a neurological disorder therapeutic agent, which directly acts on the vascular barrier breakdown caused by disappearance of claudin-5 depending upon oxygen concentration.

### Example 5

### (Examination of suppressive effect on vascular barrier breakdown by anti-ADAM12 antibody and anti-ADAM17 antibody)

Normal goat serum-derived IgG (NC: manufactured by SIGMA-ALDRICH), anti-ADAM12 antibody (Anti-ADAM12 antibody: goat anti-human ADAM12 polyclonal antibody sc-16527 (C-20): manufactured by Santa Cruz Biotechnology) and anti-ADAM17 antibody (Anti-ADAM17 antibody: goat anti-human ADAM17 polyclonal antibody sc-6416 (C-15): manufactured by Santa Cruz Biotechnology) were intravitreally administered to mice. Thereafter, in mice (normoxia) which were raised under normoxia for 24 hours and mice (hypoxia) which were raised under hypoxia (4% to 7%) for 24 hours, leakage of the tracer injected therein was observed by a confocal microscope. The tracer used herein and a method for evaluating leakage of the tracer injected by a confocal microscope are the same as described in Example 4. The results are shown in Figure 9.

In the mice to which normal serum-derived IgG was intravitreally administered, in the blood vessel in the retinal tissue placed under hypoxia, leakage of fluorescent dyes injected as a tracer was increased, compared to the blood vessel in the retinal tissue placed under normoxia. In contrast, in mice in which an anti-ADAM12 antibody or an anti-ADAM17 antibody is intravitreally administrated, in the blood vessel in the retinal tissue placed under hypoxia, an increase of fluorescent dye leakage was suppressed. Particularly, it seems that the anti-ADAM12 antibody rather than the anti-ADAM17 antibody tends to have a stronger suppressive effect on an increase of fluorescent dye leakage. Thus, it was clearly demonstrated that oxygen concentration-dependent vascular barrier breakdown can be suppressed by reducing the physiological activities of ADAM12 and ADAM17 by using an anti-ADAM12 antibody and an anti-ADAM17 antibody.

### Example 6

### (Examination on suppressive effect of vascular barrier breakdown by ADAM17 knockout mice)

Wild type mice (WT) and ADAM17 knockout mice (ADAM17^{Δ/Δ}) were raised under normoxia for 48 hours (normoxia) and under hypoxia (4% to 7%) for 48 hours (hypoxia). In these mice, leakage of the tracer injected was observed by a confocal microscope. The tracer used and the method of evaluating leakage of the tracer injected by a confocal microscope were the same as in Example 4. ADAM17 knockout mice (ADAM17^{Δ/Δ}), which were prepared in accordance with the method of Horiuchi K et al. (J Immunol (2007) 179:2686-2689), were provided by the authors of the document. The results are shown in Figure 10.

In the wild type mice, in the blood vessel in the retinal tissue placed under hypoxia, leakage of the fluorescent dyes injected as a tracer was increased compared to the blood vessel in the retinal tissue placed under normoxia. In contrast, in ADAM17 knockout mice, in the blood vessel in the retinal tissue placed under hypoxia, leakage of the fluorescent dyes injected did not increase. Thus, it was clearly demonstrated that oxygen concentration-dependent vascular barrier breakdown can be suppressed by suppressing expression of the ADAM17 gene.

### Industrial Applicability

According to the present invention, neurological disorders can be treated by directly acting on vascular barrier breakdown. Thus, the present invention is useful in the medical field.

## Claims

1. Any one of the following (a) to (c) for use in the treatment of a cerebral neurological disorder or a retinal neurological disorder caused by breakdown of a neural vascular barrier function:
(a) a functional nucleic acid against ADAM (a disintegrin and metalloproteinase) 12;
(b) a functional nucleic acid expression vector containing a DNA encoding the functional nucleic acid (a); and
(c) a neutralizing antibody against ADAM12.

2. Any one of (a) to (c) for use according to claim 1, wherein the functional nucleic acid is siRNA.

3. Any one of (a) to (c) for use according to claim 2, wherein the functional nucleic acid against ADAM12 is a siRNA consisting of a sense strand sequence of nucleotides represented by SEQ ID NO: 1 and an antisense strand sequence complementary thereto represented by SEQ ID NO: 2, or a siRNA consisting of a sense strand sequence of nucleotides represented by SEQ ID NO: 3 and an antisense strand sequence complementary thereto represented by SEQ ID NO: 4.

4. Any one of (a) to (c) for use according to any one of claims 1 to 3, wherein the cerebral neurological disorder is brain edema.

5. Any one of (a) to (c) for use according to any one of claims 1 to 3, wherein the retinal neurological disorder is retinal edema.

## Patentansprüche

1. Beliebiges der folgenden (a) bis (c) zur Verwendung bei der Behandlung einer zerebralen neurologischen Störung oder einer retinalen neurologischen Störung, die durch den Zusammenbruch einer neuronalen vaskulären Barrierefunktion verursacht wird:
(a) eine funktionelle Nukleinsäure gegen ADAM (ein Desintegrin und Metalloproteinase) 12;
(b) einen funktionellen Nukleinsäure-Expressionsvektor, der eine DNA enthält, die für die funktionelle Nukleinsäure (a) kodiert; und
(c) einen neutralisierenden Antikörper gegen ADAM12.

2. Beliebiges von (a) bis (c) zur Verwendung nach Anspruch 1, wobei die funktionelle Nukleinsäure siRNA ist.

3. Beliebiges von (a) bis (c) zur Verwendung nach Anspruch 2, wobei die funktionelle Nukleinsäure gegen ADAM12 eine siRNA, die aus einer Sense-Strang-Sequenz von Nukleotiden, wiedergegeben durch SEQ ID NR.: 1, und einer dazu komplementären Antisense-Strang-Sequenz, wiedergegeben durch SEQ ID NR.: 2, oder eine siRNA ist, bestehend aus einer Sense-Strang-Sequenz von Nukleotiden, wiedergegeben durch SEQ ID NR.: 3, und einer dazu komplementären Antisense-Strang-Sequenz, wiedergegeben durch SEQ ID NR.: 4, besteht.

4. Beliebiges von (a) bis (c) zur Verwendung nach einem der Ansprüche 1 bis 3, wobei die zerebrale neurologische Störung ein Hirnödem ist.

5. Beliebiges von (a) bis (c) zur Verwendung nach einem der Ansprüche 1 bis 3, wobei die retinale neurologische Störung ein Netzhautödem ist.

## Revendications

1. Un quelconque des (a) à (c) qui suivent destinés à être utilisés dans le traitment d'un trouble neurologique cérébral ou d'un trouble neurologique rétinien provoqué par une défaillance d'une fonction barrière neurovasculaire :
(a) un acide nucléique fonctionnel contre ADAM (une disintégrine et une métalloprotéinase) 12 ;
(b) un vecteur d'expression d'acide nucléique fonctionnel contenant un ADN codant pour l'acide nucléique fonctionnel (a) ; et
(c) un anticorps de neutralisation contre ADAM12.

2. Un quelconque des (a) et (c) destinés à être utilisés selon la revendication1, dans lequel l'acide nucléique fonctionnel est un siARN.

3. Un quelconque des (a) et (c) destinés à être utilisés selon la revendication 2, dans lequel l'acide nucléique fonctionnel contre ADAM12 est un siARN constitué par une séquence à brins sens de nucléotides représentée par SEQ ID NO : 1 et une séquence à brins antisens complémentaire à celle-ci représentée par SEQ ID NO : 2, ou un siARN constitué par une séquence à brins sens de nucléotides représentée par SEQ ID NO : 3 et une séquence à brins antisens complémentaire à celle-ci représenté par SEQ ID NO : 4.

4. Un quelconque des (a) et (c) destinés à être utilisés selon l'une quelconque des revendications 1 à 3, dans lequel le trouble neurologique cérébral est un oedème cérébral.

5. Un quelconque des (a) et (c) destinés à être utilisés selon l'une quelconque des revendications 1 à 3, dans lequel le trouble neurologique rétinien est un oedème rétinien.


### SEQUENCE LISTING

<110> Yamaguchi University
<120> Therapeutic agent for neurological disease
<130> FH25-015WO
<150> JP 2013-093071
   <151> 2013-04-25
<160> 44
<170> PatentIn version 3.5
<210> 1
   <211> 19
   <212> RNA
   <213> artificial
<220>
   <223> Inventor: Ikeda, Eiji; Cui, Dan
<220>
   <223> siRNA sequence
<400> 1
   gcuaccaccu guacucuga 19
<210> 2
   <211> 19
   <212> RNA
   <213> artificial
<220>
   <223> siRNA sequence
<400> 2
   ucagaguaca ggugguagc 19
<210> 3
   <211> 19
   <212> RNA
   <213> artificial
<220>
   <223> siRNA sequence
<400> 3
   cccuuaagau gaccaagua 19
<210> 4
   <211> 19
   <212> RNA
   <213> artificial
<220>
   <223> siRNA sequence
<400> 4
   uacuugguca ucuuaaggg 19
<210> 5
   <211> 19
   <212> RNA
   <213> artificial
<220>
   <223> siRNA sequence
<400> 5
   ggacguaauu gagcgauuu 19
<210> 6
   <211> 19
   <212> RNA
   <213> artificial
<220>
   <223> siRNA sequence
<400> 6
   aaaucgcuca auuacgucc 19
<210> 7
   <211> 19
   <212> RNA
   <213> artificial
<220>
   <223> siRNA sequence
<400> 7
   ccaggauuuu gauauggga 19
<210> 8
   <211> 19
   <212> RNA
   <213> artificial
<220>
   <223> siRNA sequence
<400> 8
   ucccauauca aaauccugg 19
<210> 9
   <211> 21
   <212> DNA
   <213> artificial
<220>
   <223> siRNA sequence DNA/RNA
<220>
   <221> misc_feature
   <222> (20)..(21)
   <223> overhang
<400> 9
   ggaacucagu uuucgaguut t 21
<210> 10
   <211> 21
   <212> DNA
   <213> artificial
<220>
   <223> siRNA sequence DNA/RNA
<220>
   <221> misc_feature
   <222> (20)..(21)
   <223> overhang
<400> 10
   aacucgaaaa cugaguucct g 21
<210> 11
   <211> 21
   <212> DNA
   <213> artificial
<220>
   <223> siRNA sequence DNA/RNA
<220>
   <221> misc_feature
   <222> (20)..(21)
   <223> overhang
<400> 11
   ggaacucagu uuucgaguut t 21
<210> 12
   <211> 21
   <212> DNA
   <213> artificial
<220>
   <223> siRNA sequence DNA/RNA
<220>
   <221> misc_feature
   <222> (20)..(21)
   <223> overhang
<400> 12
   aacucgaaaa cugaguucct g 21
<210> 13
   <211> 21
   <212> DNA
   <213> artificial
<220>
   <223> siRNA sequence DNA/RNA
<220>
   <221> misc_feature
   <222> (20)..(21)
   <223> overhang
<400> 13
   cagaaauccu aucaauauat t 21
<210> 14
   <211> 21
   <212> DNA
   <213> artificial
<220>
   <223> siRNA sequence DNA/RNA
<220>
   <221> misc_feature
   <222> (20)..(21)
   <223> overhang
<400> 14
   uauauugaua ggauuucugt c 21
<210> 15
   <211> 21
   <212> DNA
   <213> artificial
<220>
   <223> siRNA sequence DNA/RNA
<220>
   <221> misc_feature
   <222> (20)..(21)
   <223> overhang
<400> 15
   cguccauugu ugcucaugat t 21
<210> 16
   <211> 21
   <212> DNA
   <213> artificial
<220>
   <223> siRNA sequence DNA/RNA
<220>
   <221> misc_feature
   <222> (20)..(21)
   <223> overhang
<400> 16
   ucaugagcaa caauggacgc a 21
<210> 17
   <211> 21
   <212> DNA
   <213> artificial
<220>
   <223> siRNA sequence DNA/RNA
<220>
   <221> misc_feature
   <222> (20)..(21)
   <223> overhang
<400> 17
   gacuucuccg gaauccguat t 21
<210> 18
   <211> 21
   <212> DNA
   <213> artificial
<220>
   <223> siRNA sequence DNA/RNA
<220>
   <221> misc_feature
   <222> (20)..(21)
   <223> overhang
<400> 18
   uacggauucc ggagaaguct g 21
<210> 19
   <211> 21
   <212> DNA
   <213> artificial
<220>
   <223> siRNA sequence DNA/RNA
<220>
   <221> misc_feature
   <222> (20)..(21)
   <223> overhang
<400> 19
   cauuacuguu cagaacuaut t 21
<210> 20
   <211> 21
   <212> DNA
   <213> artificial
<220>
   <223> siRNA sequence DNA/RNA
<220>
   <221> misc_feature
   <222> (20)..(21)
   <223> overhang
<400> 20
   auaguucuga acaguaauga t 21
<210> 21
   <211> 21
   <212> DNA
   <213> artificial
<220>
   <223> siRNA sequence DNA/RNA
<220>
   <221> misc_feature
   <222> (20)..(21)
   <223> overhang
<400> 21
   gcuaccaccu guacucugat t 21
<210> 22
   <211> 21
   <212> DNA
   <213> artificial
<220>
   <223> siRNA sequence DNA/RNA
<220>
   <221> misc_feature
   <222> (20)..(21)
   <223> overhang
<400> 22
   ucagaguaca ggugguagcg t 21
<210> 23
   <211> 21
   <212> DNA
   <213> artificial
<220>
   <223> siRNA sequence DNA/RNA
<220>
   <221> misc_feature
   <222> (20)..(21)
   <223> overhang
<400> 23
   cccuuaagau gaccaaguat t 21
<210> 24
   <211> 21
   <212> DNA
   <213> artificial
<220>
   <223> siRNA sequence DNA/RNA
<220>
   <221> misc_feature
   <222> (20)..(21)
   <223> overhang
<400> 24
   uacuugguca ucuuaagggt c 21
<210> 25
   <211> 21
   <212> DNA
   <213> artificial
<220>
   <223> siRNA sequence DNA/RNA
<220>
   <221> misc_feature
   <222> (20)..(21)
   <223> overhang
<400> 25
   gauggacccu guugucaaat t 21
<210> 26
   <211> 21
   <212> DNA
   <213> artificial
<220>
   <223> siRNA sequence DNA/RNA
<220>
   <221> misc_feature
   <222> (20)..(21)
   <223> overhang
<400> 26
   uuugacaaca ggguccauca g 21
<210> 27
   <211> 21
   <212> DNA
   <213> artificial
<220>
   <223> siRNA sequence DNA/RNA
<220>
   <221> misc_feature
   <222> (20)..(21)
   <223> overhang
<400> 27
   cagcacaacc ugauagaaat t 21
<210> 28
   <211> 21
   <212> DNA
   <213> artificial
<220>
   <223> siRNA sequence DNA/RNA
<220>
   <221> misc_feature
   <222> (20)..(21)
   <223> overhang
<400> 28
   uuucuaucag guugugcugg g 21
<210> 29
   <211> 21
   <212> DNA
   <213> artificial
<220>
   <223> siRNA sequence DNA/RNA
<220>
   <221> misc_feature
   <222> (20)..(21)
   <223> overhang
<400> 29
   ggacguaauu gagcgauuut t 21
<210> 30
   <211> 21
   <212> DNA
   <213> artificial
<220>
   <223> siRNA sequence DNA/RNA
<220>
   <221> misc_feature
   <222> (20)..(21)
   <223> overhang
<400> 30
   aaaucgcuca auuacgucct g 21
<210> 31
   <211> 21
   <212> DNA
   <213> artificial
<220>
   <223> siRNA sequence DNA/RNA
<220>
   <221> misc_feature
   <222> (20)..(21)
   <223> overhang
<400> 31
   ccaggauuuu gauaugggat t 21
<210> 32
   <211> 21
   <212> DNA
   <213> artificial
<220>
   <223> siRNA sequence DNA/RNA
<220>
   <221> misc_feature
   <222> (20)..(21)
   <223> overhang
<400> 32
   ucccauauca aaauccuggt a 21
<210> 33
   <211> 21
   <212> DNA
   <213> artificial
<220>
   <223> siRNA sequence DNA/RNA
<220>
   <221> misc_feature
   <222> (20)..(21)
   <223> overhang
<400> 33
   cuccuucuuu gagacggaat t 21
<210> 34
   <211> 21
   <212> DNA
   <213> artificial
<220>
   <223> siRNA sequence DNA/RNA
<220>
   <221> misc_feature
   <222> (20)..(21)
   <223> overhang
<400> 34
   uuccgucuca aagaaggagg t 21
<210> 35
   <211> 21
   <212> DNA
   <213> artificial
<220>
   <223> siRNA sequence DNA/RNA
<220>
   <221> misc_feature
   <222> (20)..(21)
   <223> overhang
<400> 35
   ccaacgcagu aucuauugat t 21
<210> 36
   <211> 21
   <212> DNA
   <213> artificial
<220>
   <223> siRNA sequence DNA/RNA
<220>
   <221> misc_feature
   <222> (20)..(21)
   <223> overhang
<400> 36
   ucaauagaua cugcguugga t 21
<210> 37
   <211> 21
   <212> DNA
   <213> artificial
<220>
   <223> siRNA sequence DNA/RNA
<220>
   <221> misc_feature
   <222> (20)..(21)
   <223> overhang
<400> 37
   gcgcaucuca uaauugcuat t 21
<210> 38
   <211> 21
   <212> DNA
   <213> artificial
<220>
   <223> siRNA sequence DNA/RNA
<220>
   <221> misc_feature
   <222> (20)..(21)
   <223> overhang
<400> 38
   uagcaauuau gagaugcgct t 21
<210> 39
   <211> 21
   <212> DNA
   <213> artificial
<220>
   <223> siRNA sequence DNA/RNA
<220>
   <221> misc_feature
   <222> (20)..(21)
   <223> overhang
<400> 39
   gcccauaucu ucauuagaat t 21
<210> 40
   <211> 21
   <212> DNA
   <213> artificial
<220>
   <223> siRNA sequence DNA/RNA
<220>
   <221> misc_feature
   <222> (20)..(21)
   <223> overhang
<400> 40
   uucuaaugaa gauaugggct g 21
<210> 41
   <211> 21
   <212> DNA
   <213> artificial
<220>
   <223> siRNA sequence DNA/RNA
<220>
   <221> misc_feature
   <222> (20)..(21)
   <223> overhang
<400> 41
   ggaggcuuuu guuuccguat t 21
<210> 42
   <211> 21
   <212> DNA
   <213> artificial
<220>
   <223> siRNA sequence DNA/RNA
<220>
   <221> misc_feature
   <222> (20)..(21)
   <223> overhang
<400> 42
   uacggaaaca aaagccuccg t 21
<210> 43
   <211> 21
   <212> DNA
   <213> artificial
<220>
   <223> siRNA sequence DNA/RNA
<220>
   <221> misc_feature
   <222> (20)..(21)
   <223> overhang
<400> 43
   gaaguacuuu caacgacuat t 21
<210> 44
   <211> 21
   <212> DNA
   <213> artificial
<220>
   <223> siRNA sequence DNA/RNA
<220>
   <221> misc_feature
   <222> (20)..(21)
   <223> overhang
<400> 44
   uagucguuga aaguacuuct a 21